# EUROPEAN PATENT SPECIFICATION

(11) **EP 1 490 455 B1**
(45) Date of publication and mention of the grant of the patent: **25.07.2007**
(21) Application number: 02714411.2
(22) Date of filing: 27.03.2002
(51) Int. Cl.: C10C 1/08, C07C 15/30, B01D 3/14, C07C 7/04

(54) **A PROCESS FOR OBTAINING HIGH PURITY PHENANTHRENE AND A SYSTEM THEREOF**
VERFAHREN ZUR GEWINNUNG VON PHENANTHREN HOHER REINHEIT UND VORRICHTUNG DAFÜR
SYSTEME ET PROCEDE D'OBTENTION DE PHENANTHRENE DE GRANDE PURETE

(43) Date of publication of application: 29.12.2004
(73) Proprietor: Council of Scientific and Industrial Research;an Indian Reg. body incorporated under Reg. of Societies Act (Act XXI of 1860), New Delhi 110 001 (IN)
(72) Inventor: TIWARI,Kaushal,Kishore, P.O. & Dist: DHANBAD, Jharkhand (IN); RAO,Sukuru,Ramakrishna Central Fuel Research Inst., Jharkhand (IN); THAKUR,Sanjay,Kumar Central Fuel Research Inst., Jharkhand (IN); BANERJI, Somnath Central Fuel Research Institute, Jharkhand (IN)
(74) Representative: Long, Giorgio
(86) International application number: PCT/IN2002/000076
(87) International publication number: WO 2003/080767

(56) References cited:
- DE-C- 552 586
- GB-A- 801 907
- US-A- 2 590 096
- US-A- 2 795 538
- "Ullmann's Encyclopedia of Industrial Chemistry" 1989 , VCH VERLAGSGESELLSCHAFT MBH , WEINHEIM (FRG) XP002223002 13 page 270, column 1, line 3 -page 271, column 1, line 24

## Description

### Field of the invention

The present invention relates to a process to obtain about 95% purity Phenanthrene and a system for obtaining the said product from coal tar distilled fraction containing crude phenanthrene.

### Background and prior art references

Phenanthrene forms the basis for production of 9,10-phenanthraquinone and 2,2'-diphenic acid. It can also be used to synthesize anthracene via the isomerisation product of sym-octahydrophenanthrene. Electrically conductive substances, e.g., for use in batteries and solar cells, can be produced by the electrochemical conversion of phenanthrene diazonium salts in a solvent containing a conductive salt and subsequent doping with various ions (like sodium, barium, hydrogen etc). Liquid crystalline 7-n-alkyl-9, 10-dihydrophenanthrene-2-carboxylic acid ester, used for optical-electronic applications, can be synthesized from 9,10-dihydrophenanthrene. By cross-linking with p-xylene glycol and 4-toluenesulphonic acid, poly-condensed thermosetting resins are obtained for composites or temperature-resistant, electrically insulating coatings. A polyamide-polimide resin can be produced by oxidation of phenanthrene to phenanthrene-9,10-quinone and 9,10-diol, condensation with formaldehyde, oxidation to the polycarboxylic acid, formation of the anhydride and finally reaction with an aromatic diamine. This resin is suitable for use in high temperature insulators, printed circuit boards, and laminates. Phenanthrene has been proposed as a plasticizer for plastics and molding compounds; phenanthrene and alkylphenantherenes have been suggested as stabilizers for mineral oil products.

Phenanthrene, at a concentration of 5%, is the second most important coal tar constituent in terms of quantity after naphthalene. During primary distillation of coal tar, it is concentrated in the anthracene oil fraction. After crystallization of the anthracene residues, the phenanthrene is recovered as a fraction from the filtrate of this crystallization, or from the top fraction of crude anthracene distillation, by re-distillation. Technically pure grades of phenanthrene are obtained by sulfuric acid refining and re-crystallization from methanol, or by repeated rectification of the phenanthrene fraction. The accompanying substances can be separated either by partial sulphonation, or by partial condensation with formaldehyde and hydrogen chloride. Detailed search on patent databases and other literature did not result in any relevant reference.

GB 801,907 describes a process for the continuous production by distillation of high-boiling components of coal-tar, which become highly concentrated in high-boiling coal tar fractions, in order to facilitate further processing, for example, the recovery of pure products such as anthracene, carbazole or pyrene, and increase the yields of these ingredients.

DE 552586 describes a process for the production of solid hydrocarbons from tars, which consists in splitting the crude tars into fractions which contain mainly the several solid hydrocarbons, causing the solid hydrocarbons to set by cooling the fractions, separating the said solid hydrocarbons by crystallisation from the liquid hydrocarbons, and then redistilling the resultant solid hydrocarbons.

### Objects of the invention

The main object of the invention is to provide a process for obtaining high purity phenanthrene from crude phenanthrene, which obviates the drawbacks as detailed above.

Another object of the invention is to extract acenaphthene, carbazole and fluorene contained in the crude phenanthrene fraction.

Still another object of the invention is to provide a system for the enhancement of purity of phenanthrene.

### Summary of the Invention

Accordingly the present invention provides a process and a system for obtaining phenanthrene of about 95% purity from coal tar distilled fraction containing crude phenanthrene according to the appended claims

### Brief description of the accompanying drawings

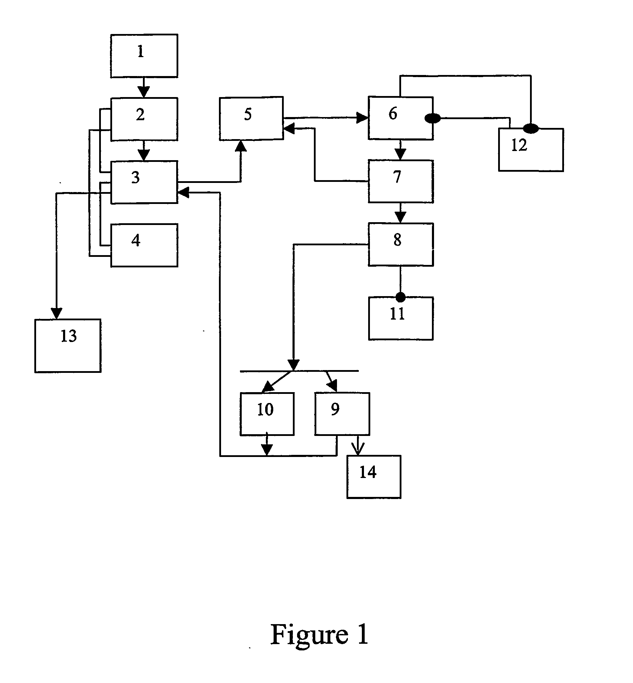

**Figure 1** represents a flow chart of obtaining pure phenanthrene, wherein
1 Feed Pump
2 Pre-heater
3 Kettle type re-boiler
4 Thermic boiler
5 Distillation column
6 Condenser
7 Hot Catch Pot
8 Receiver
9 Product of 2^{nd} Cut receiving means
10 Product of 1^{st} Cut receiving means
11 Vacuum Pump
12 Heat generating means
13 Heavy Product receiving means
14 Receiving means for receiving pure phenanthrene

### Detailed Description of the Invention

The invention provides a process from which 95% pure phenanthrene is obtained, in addition, other products like acenaphthene, flourene, anthracene and carbazole are being separated from the crude phenanthrene.

The invention relates to a system, which is having continuous circulation of hot-thermic oil from thermic boiler (4) to kettle re-boiler (3) thereby to pre-heater (2) and finally back to thermic boiler (4) leading to conservation of heat.

The heat generated in condenser (6) can be sent to a heat generating means (12) thereby recovering heat.

According to the invention the heat recovery means is a hot water generation means (12), which prevents solidification of product and thereby avoiding choking of the condenser (6).

A portion of the product of hot catch pot (7) is sent to distillation column (5) for re-distillation.

Yet another embodiment of the invention provides a system, in which the first cut product from the receiving means (10) can be sent to the kettle type boiler (3) for carrying out re-distillation.

Yet another embodiment, the phenanthrene obtained has a purity of 95% which is recovered from the recovery means (14).

Yet another embodiment of the invention provides separation .of acenaphthene, flourene, anthracene and carbazole from crude phenanthrene.

The system of the invention allows modifying the parameters of temperature and pressure.

Another embodiment of the invention, the percentage yields of various products as follows.
Lighter products - Acenaphthene and fluorene about 15 to 18 %
Carbazole and other heavy products about 27 to 33%
Phenanthrene about 50 - 55 %.

Acenaphthene and fluorene, the lighter products are collected at (10)

Product from hot catch pot (7) is refluxed to (5) for developing the purity of phenanthrene.

From distillation, column (5), heavier fractions remains at bottom whereas the lighter fractions goes as condensate to (6) and (7). This process is continued several times to get purity of product,

Hot catch pot (7) has been partitioned into two and valves control collections at the parts.

One part goes to (8) as the main product whereas other part is refluxed to distillation cloumn (5) for re-distillation. The objective of this to obtain pure phenanthrene as much as possible.

The differentiation of fractions at distillation column (5) is done by the observance of temperature at (5). For first distillation, if the temperature is maintained in between 190 to 214°C, then it is sent to (10) and if it is in between 214-228°C, it is sent to (9). In case of re-distillation of product at (9), the temperature is maintained in between 215-223°C. Controlling valves performs the above operations.

The heavy products are carbazole, flourenthrene and other tarry products, which are obtained above 400°C. Practically the heavier products are not taken into consideration. From kettle type re-boiler (3), after superheated vapors are transferred to (5) for redistillation, the residue is collected at (13).

The function of the vacuum pump (11) is to create lower vapor pressure, thereby lowering boiling points of various fractions.

Steam created at (12) is circulated to condenser (6) and the same is re-circulated again and again from 6→ to 12, 12→6 etc,. the purpose of steam is to prevent the condenser from choking as melting point of phenanthrene is 101°C.

The temperature maintained at different points are:
(2) 150 to 200°C;
(3) 230-250°C;
(4) 250-300°C;
(5) 224-230°C;

Hot oil is circulated between (3), (2), (4) and (3).

The novel features of the invention are:
1. Phenanthrene of 95% purity could be obtained from crude phenanthrene, the literature for which is not available elsewhere.
2. The system, has the provision to use steam generated from the condenser (6) for the distillation of the entire fractions thereby choking of pipelines could be avoided by this construction.

The following examples are given by way of illustration of the present invention and should not be construed to limit the scope of the present invention.

### Example-1

1100 gms of crude phenanthrene was subjected to distillation. The various product breakups are indicated without taking into account of other residues. The first cut was distilled in the range of 190 to 214 degree Celsius wherein 130 gms of the product (43.53 gms of Phenanthrene, 9.364 gms of Acenaphthene; 72.605 gms of fluorene) was obtained. The second cut was distilled in the range of 220 to 232 degree Celsius wherein 788 gms of the product (727.311gms of Phenanthrene, 8.97gms of Carbazole and 36.48 gms of Acenaphthene) was obtained. From the bottom part, 175 gms of the product (94.46 gms Phenanthrene and 80.54gms of Carbazole) was obtained. The residue of the second cut was 753 gms and was subjected to re-distillation; the first cut was distilled in the temperature range of 220 to 223 degree Celsius from which 118 gms of the product (23.53 gms of Acenaphthene, 71.97gms of Phenanthrene and 0.82 gms of Carbazole) was obtained; the second cut was distilled in the temperature range of 224 to 230 degree Celsius from which 531 gms of the product (516 gms of Phenanthrene, 5.99 gms of Acenaphthene and 8.03 gms of Fluorene) was obtained; distillation of bottom part yielded 103 gms of the product (0.193 gms of Fluorene, 68.04 gms of Phenanthrene and 34.76 gms of carbazole) was obtained

The main advantages of the present invention are:
1. Recycling of different cuts could separate acenaphthene, fluorene and carbazole, which are also useful chemicals.
2. The chemicals and solvents used in the process are very cheap; thereby the process is cost effective.

## Claims

1. A process for obtaining phenanthrene of about 95% purity from coal tar distilled fraction containing crude phenanthrene by performing fractional distillation at a reduced pressure comprising the step of providing a system comprising:
a) a feed pump (1),
b) a pre-heater (2) directly downstream of the feed pump (1),
c) a kettle-type re-boiled (3) directly downstream to the pre-heater (2) for obtaining superheated vapour,
d) a thermic boiler (4) connected to the kettle type reboiler (3) for circulating hot termic oil from thermic boiler (4) to kettle reboiler (3) thereby to pre-heater (2) and finally back to thermic boiler (4) to conserve heat,
e) a distillation column (5) directly linked with an upper part of the kettle type reboiler (3),
f) a condenser (6), directly in fluid communication with the distillation column (5), wherein the condenser (6) is connected to a hot water generation means (12), wherein the steam generated at said generation means (12) is circulated to the condenser (6) thus preventing the condenser (6) from choking.
g) a hot catch pot (7) directly down stream of the condenser (6),
h) a conduit connecting the hot catch Dot (7) to the distillation column (5),
i) a first receiving means (8) directly down stream to the hot catch pot (7) with a vacuum pump (11);
j) a second receiving means (9) and a third receiving means (10) having disconnectable fluid communication with the first receiving means (8).
k) a fourth receiving means (13) in fluid communication with a lower part of the kettle type reboiler,
l) a fifth receiving means (14) in direct communication with second receiving means (9), for receiving pure phenanthrene,
m) a conduit leading from the second receiving means (9) and the third receiving means (10), to the kettle type rerboiler (3) for carrying out redistillation,
wherein the differentiation of fractions at distillation column (5) is done by observing the following steps:
- first distilled fraction is achieved between 190 and 214 °C and it is sent to the third receiving means (10) :
- second distilled fraction is achieved between 214 and 228°C and it is sent to the second receiving means (9).
- redistilling the product contained in the second receiving means (9) at a temperature between 215 and 223°C
- refluxing a portion of the product from the hot catch pot 7 to the distillation column (5) for developing the purity of phenanthrene.

2. A process as claimed in claim 1, wherein the phenanthrene obtained has a purity of 95%.

3. A process as claimed in claim 1, wherein acenaphthene, flourene, anthracene and carbazole are separated from the crude phenanthrene.

4. A system for obtaining about 95 % pure phenanthrene from coal tar distilled fraction, said system comprising of :
a) a feed pump (1),
b) a pre-heater (2) directly downstream of the feed pump (1),
c) a kettle-type re-boiled (3) directly downstream to the pre-heater (2) for obtaining superheated vapour,
d) a thermic boiler (4) connected to the kettle type reboiler (3) for circulating hot termic oil from thermic boiler (4) to kettle reboiler (3) thereby to pre-heater (2) and finally back to thermic boiler (4) to conserve heat,
e) a distillation column (5) directly linked with an upper part of the kettle type reboiler (3),
f) a condenser (6), directly in fluid communication with the distillation column (5), wherein the condenser (6) is connected to a hot water generation means (12), wherein the steam generated at said generation means (12) is circulated to the condenser (6) thus preventing the condenser (6) from choking.
g) a hot catch pot (7) directly down stream of the condenser (6),
h) a conduit connecting the hot catch pot (7) to the distillation column (5)
i) a first receiving means (8) directly down stream to the hot catch pot (7) with a vacuum pump (11);
j) a second receiving means (9) and a third receiving means (10) having disconnectable fluid communication with the first receiving means (8).
k) a fourth receiving means (13) in fluid communication with a lower part of the kettle type reboiler,
l) a fifth receiving means (14) in direct communication with second receiving means (9), for obtaining pure phenanthrene,
m) a conduit leading from the second receiving means (9) and the third receiving means (10), to the kettle type rerboiler (3) for carrying out redistillation.

5. A system as claimed in claim 4, wherein the third receiving means (10) is in communication with the kettle type boiler (3).

6. A system as claimed in claim 4, wherein the system allows modifying the parameters of temperature and pressure.

## Patentansprüche

1. Verfahren zur Gewinnung von Phenanthren von etwa 95 % Reinheit aus einer rohes Phenanthren enthaltenden destillierten Fraktion von Kohleteer durch Durchführen einer fraktionierten Destillation unter vermindertem Druck, das die Stufe der Bereitstellung eines Systems umfasst, das
a) eine Beschickungspumpe (1),
b) einen Vorerhitzer (2) direkt stromabwärts der Beschickungspumpe (1),
c) einen Reboiler des Kesseltyps (3) direkt stromabwärts des Vorerhitzers (2) zur Gewinnung von überhitztem Dampf,
d) einen Heizdampferzeuger (4), der mit dem Reboiler des Kesseltyps (3) zur Zirkulation von heißem Heizöl von dem Heizdampferzeuger (4) zum Kesselreboiler (3) und **dadurch** zum Vorerhitzer (2) und schließlich zurück zum Heizdampferzeuger (4) zur Wärmekonservierung verbunden ist,
e) eine Destillationskolonne (5), die direkt mit einem oberen Teil des Reboilers des Kesseltyps (3) verbunden ist,
f) einen Kühler (6), der direkt in Fluidkommunikation mit der Destillationskolonne (5) steht, wobei der Kühler (6) mit einem Heißwassererzeugungsmittel (12) verbunden ist, wobei der an dem Erzeugungsmittel (12) erzeugte Wasserdampf zu dem Kühler (6) zirkuliert wird, um ein Verstopfen des Kühlers (6) zu verhindern,
g) einen Heißabscheider (7) direkt stromabwärts des Kühlers (6),
h) eine Leitung, die den Heißabscheider (7) mit der Destillationskolonne (5) verbindet,
i) ein erstes Aufnahmemittel (8) direkt stromabwärts des Heißabscheiders (7) mit einer Vakuumpumpe (11),
j) ein zweites Aufnahmemittel (9) und ein drittes Aufnahmemittel (10) mit unterbrechbarer Fluidkommunikation mit dem ersten Aufnahmemittel (8),
k) ein viertes Aufnahmemittel (13) in Fluidkommunikation mit einem unteren Teil des Reboilers des Kesseltyps,
l) ein fünftes Aufnahmemittel (14) in direkter Kommunikation mit dem zweiten Aufnahmemittel (9) zur Aufnahme von reinem Phenanthren,
m) eine Leitung, die von dem zweiten Aufnahmemittel (9) und dem dritten Aufnahmemittel (10) zu dem Reboiler des Kesseltyps (3) zur Durchführung einer Redestillation führt, umfasst,
wobei die Differenzierung der Fraktionen an der Destillationskolonne (5) durch Beachten der im folgenden angegebenen Stufen erfolgt:
- die erste destillierte Fraktion wird zwischen 190 und 214 °C erhalten und zu dem dritten Aufnahmemittel (10) geschickt,
- die zweite destillierte Fraktion wird zwischen 214 und 228 °C erhalten und zu dem zweiten Aufnahmemittel (9) geschickt,
- Redestillation des in dem zweiten Aufnahmemittel (9) enthaltenen Produkts bei einer Temperatur zwischen 215 und 223 °C,
- Refluxieren eines Teils des Produkts aus dem Heißabscheider (7) in die Destillationskolonne (5) zur Entwicklung der Reinheit von Phenanthren.

2. Verfahren nach Anspruch 1, wobei das erhaltene Phenanthren eine Reinheit von 95 % aufweist.

3. Verfahren nach Anspruch 1, wobei Acenaphthen, Fluoren, Anthracen und Carbazol von dem rohen Phenanthren abgetrennt werden.

4. System zur Gewinnung von etwa 95 % reinem Phenanthren aus einer destillierten Fraktion von Kohleteer, wobei das System
a) eine Beschickungspumpe (1),
b) einen Vorerhitzer (2) direkt stromabwärts der Beschickungspumpe (1),
c) einen Reboiler des Kesseltyps (3) direkt stromabwärts des Vorerhitzers (2) zur Gewinnung von überhitztem Dampf,
d) einen Heizdampferzeuger (4), der mit dem Reboiler des Kesseltyps (3) zur Zirkulation von heißem Heizöl von dem Heizdampferzeuger (4) zum Kesselreboiler (3) und **dadurch** zum Vorerhitzer (2) und schließlich zurück zum Heizdampferzeuger (4) zur Wärmekonservierung verbunden ist,
e) eine Destillationskolonne (5), die direkt mit einem oberen Teil des Reboilers des Kesseltyps (3) verbunden ist,
f) einen Kühler (6), der direkt in Fluidkommunikation mit der Destillationskolonne (5) steht, wobei der Kühler (6) mit einem Heißwassererzeugungsmittel (12) verbunden ist, wobei der an dem Erzeugungsmittel (12) erzeugte Wasserdampf zu dem Kühler (6) zirkuliert wird, um ein Verstopfen des Kühlers (6) zu verhindern,
g) einen Heißabscheider (7) direkt stromabwärts des Kühlers (6),
h) eine Leitung, die den Heißabscheider (7) mit der Destillationskolonne (5) verbindet,
i) ein erstes Aufnahmemittel (8) direkt stromabwärts des Heißabscheiders (7) mit einer Vakuumpumpe (11),
j) ein zweites Aufnahmemittel (9) und ein drittes Aufnahmemittel (10) mit unterbrechbarer Fluidkommunikation mit dem ersten Aufnahmemittel (8),
k) ein viertes Aufnahmemittel (13) in Fluidkommunikation mit einem unteren Teil des Reboilers des Kesseltyps,
l) ein fünftes Aufnahmemittel (14) in direkter Kommunikation mit dem zweiten Aufnahmemittel (9) zur Gewinnung von reinem Phenanthren,
m) eine Leitung, die von dem zweiten Aufnahmemittel (9) und dem dritten Aufnahmemittel (10) zu dem Reboiler des Kesseltyps (3) zur Durchführung einer Redestillation führt, umfasst.

5. System nach Anspruch 4, wobei das dritte Aufnahmemittel (10) in Kommunikation mit dem Dampferzeuger des Kesseltyps (3) steht.

6. System nach Anspruch 4, wobei das System eine Modifizierung der Parameter Temperatur und Druck erlaubt.

## Revendications

1. Procédé pour obtenir du phénanthrène pur à environ 95 % à partir d'une fraction distillée de goudron contenant du phénanthrène brut en réalisant une distillation fractionnée sous pression réduite comprenant l'étape consistant à fournir un système comprenant :
a) une pompe d'alimentation (1),
b) un système de préchauffage (2) directement en aval de la pompe d'alimentation (1),
c) un rebouilleur de type bouilloire (3) directement en aval du système de préchauffage (2) pour obtenir de la vapeur surchauffée,
d) une chaudière thermique (4) raccordée au rebouilleur de type bouilloire (3) pour faire circuler l'huile thermique chaude à partir d'une chaudière thermique (4) jusqu'au rebouilleur de type bouilloire (3) et de là, jusqu'au système de préchauffage (2) et enfin de nouveau à la chaudière thermique (4) pour conserver la chaleur,
e) une colonne de distillation (5) directement liée à une partie supérieure du rebouilleur de type bouilloire (3),
f) un condensateur (6), directement en communication fluidique avec la colonne de distillation (5), dans lequel le condensateur (6) est raccordé à un moyen de génération d'eau chaude (12), dans lequel la vapeur générée audit moyen de génération (12) est mise en circulation jusqu'au condensateur (6) en évitant ainsi que le condensateur (6) ne se bloque.
g) un récipient de récupération à chaud (7) directement en aval du condensateur (6),
h) un conduit raccordant le récipient de récupération à chaud (7) à la colonne de distillation (5),
i) un premier moyen de réception (8) directement en aval du sécheur chaud (7) avec une pompe à vide (11);
j) un second moyen de réception (9) et un troisième moyen de réception (10) ayant une communication fluidique pouvant être déconnectée avec le premier moyen de réception (8).
k) un quatrième moyen de réception (13) en communication fluidique avec une partie inférieure du rebouilleur de type bouilloire,
l) un cinquième moyen de réception (14) en communication directe avec le second système de réception (9) pour recevoir du phénanthrène pur,
m) un conduit partant du second moyen de réception (9) et du troisième moyen de réception (10) jusqu'au rebouilleur de type bouilloire (3) pour réaliser la distillation,
dans lequel la différenciation des fractions au niveau de la colonne de distillation (5) est réalisée en observant les phases suivantes :
- la première fraction distillée est obtenue entre 190 et 214 °C et elle est envoyée au troisième moyen de réception (10) ;
- la seconde fraction distillée est obtenue entre 214 et 228 °C et elle est envoyée au second moyen de réception (9) ;
- la redistillation du produit contenu dans le second moyen de réception (9) s'effectue à une température comprise entre 215 et 223 °C
- une partie du produit est refluée à partir du sécheur chaud (7) à la colonne de distillation (5) pour développer la pureté du phénanthrène.

2. Procédé selon la revendication 1, dans lequel le phénanthrène obtenu a une pureté de 95 %.

3. Procédé selon la revendication 1, dans lequel l'acénaphtène, le fluorène, l'anthracène, et le carbazole sont séparés du phénanthrène brut.

4. Système pour obtenir du phénanthrène pur à environ 95 % à partir de la fraction distillée de goudron, ledit système comprenant :
a) une pompe d'alimentation (1)
b) un système de préchauffage (2) directement en aval de la pompe d'alimentation (1).
c) un rebouilleur de type bouilloire (3) directement en aval du système de préchauffage (2) pour obtenir de la vapeur surchauffée,
d) une chaudière thermique (4) raccordée au rebouilleur de type bouilloire (3) pour faire circuler l'huile thermique chaude à partir d'une chaudière thermique (4) jusqu'au rebouilleur de type bouilloire (3) et de là, jusqu'au système de préchauffage (2) et enfin de nouveau à la chaudière thermique (4) pour conserver la chaleur,
e) une colonne de distillation (5) directement liée à une partie supérieure du rebouilleur de type bouilloire (3),
f) un condensateur (6), directement en communication fluidique avec la colonne de distillation (5), dans lequel le condensateur (6) est raccordé à un moyen de génération d'eau chaude (12), dans lequel la vapeur générée audit moyen de génération (12) est mise en circulation jusqu'au condensateur (6) en évitant ainsi que le condensateur (6) ne se bloque.
g) un récipient de récupération à chaud (7) directement en aval du condensateur (6),
h) un conduit raccordant le récipient de récupération à chaud (7) à la colonne de distillation (5),
i) un premier moyen de réception (8) directement en aval du récipient de récupération à chaud (7) avec une pompe à vide (11) ;
j) un second moyen de réception (9) et un troisième moyen de réception (10) ayant une communication fluidique pouvant être déconnectée avec le premier moyen de réception (8),
k) un quatrième moyen de réception (13) en communication fluidique avec une partie inférieure du rebouilleur de type bouilloire,
l) un cinquième moyen de réception (14) en communication directe avec le second moyen de réception (9) pour obtenir du phénanthrène pur,
m) un conduit partant du second moyen de réception (9) et du troisième moyen de réception (10) jusqu'au rebouilleur de type bouilloire (3) pour réaliser la distillation.

5. Système selon la revendication 4, dans lequel le troisième moyen de réception (10) est en communication avec la chaudière de type bouilloire (3).

6. Système selon la revendication 4, dans lequel le système permet de modifier les paramètres de température et de pression.
